# EUROPÄISCHE PATENTANMELDUNG

(11) **EP 2 123 321 A1**
(43) Veröffentlichungstag der Anmeldung: **25.11.2009**
(21) Anmeldenummer: 09006856.0
(22) Anmeldetag: 20.05.2009
(51) Int. Cl.: A61M 16/10, A61M 16/16, A61M 16/20

(54) **Beatmungsgaskonditionierungseinrichtung, Teile davon und Verfahren zur Konditionierung von Beatmungsgas**

(30) Priorität: 23.05.2008 DE 102008024728
(71) Anmelder: WILAmed GmbH, 91126 Kammerstein (DE)
(72) Erfinder: Lanfranchi, Marcello, 91126 Schwabach (DE)
(74) Vertreter: Klunker . Schmitt-Nilson . Hirsch

(57) **Zusammenfassung**

Eine erfindungsgemäße Beatmungsgaskonditionierungseinrichtung weist eine Konditionierungskammer (29) auf, welcher von einer Beatmungsgasquelle Beatmungsgas zugeführt wird, das in der Konditionierungskammer (29) konditioniert, das heißt auf vorbestimmte Weise erwärmt und befeuchtet, und dann an einen zu beatmenden Patienten (15) abgegeben wird. Die Konditionierungskammer (29) ist zur Befüllung mit einer das Beatmungsgas befeuchtenden Flüssigkeit ausgebildet. Sie weist einen Kammereinlass (37) auf, der zur Verbindung mit der Beatmungsgasquelle ausgebildet ist. Sie besitzt einen Kammerauslass (43), der zur Verbindung mit dem Patienten (15) ausgebildet ist. In der Konditionierungskammer (29) ist zur Erwärmung der Flüssigkeit mit einer Flüssigkeitsheizeinrichtung versehen. Außerdem ist eine Sprudelungseinrichtung vorgesehen, die derart ausgebildet und in der Konditionierungskammer (29) derart angeordnet ist, dass sie mindestens einen Teil des von der Beatmungsgasquelle kommenden Beatmungsgases in mindestens einen Teil des Volumens der in der Konditionierungskammer (29) befindlichen Flüssigkeit zur Erwärmung und Befeuchtung des Beatmungsgases mithilfe dieses Flüssigkeitsvolumens hineinsprudelt.

## Beschreibung

Beim normalem Atmen wird die Atemluft auf ihrem Weg durch Nase, Rachen und Luftröhre konditioniert, d.h. erwärmt und befeuchtet. Etwa drei Viertel der Wärme und Feuchtigkeit werden der Atemluft von den Schleimhäuten im Nasen-RachenRaum zugefügt, das restliche Viertel in der Luftröhre. An der Luftröhrengabelung im Lungenbereich ist ein "Isothermische Sättigungsgrenze" genannter Gleichgewichtszustand von Wärme und Feuchtigkeit erreicht. Dort hat die eingeatmete Luft eine Temperatur von 37°C und 100% relative Luftfeuchtigkeit, ist also mit Wasserdampf gesättigt und enthält 44 mg Wasserdampf pro Liter Luft, womit die unteren Atemwege befeuchtet werden. Durch diese natürliche Aufbereitung der Atemluft werden die Lungenflügel gegen Austrocknung geschützt.

Im klinischen Bereich, z. B. während der Anästhesie bei einer Operation oder bei nicht ausreichender Eigenatmungsfähigkeit eines Patienten kann eine aktive Beatmung des Patienten mittels eines Beatmungsgerätes erforderlich sein. Die aktive Beatmung kann nicht-invasiv, d.h. über eine Atemmaske, oder invasiv, d.h. über Intubation mit Trachealkanüle oder über Mund- oder Nasensonde, erfolgen. Während bei nicht-invasiver Beatmung eine Voll- oder Teilkonditionierung des Beatmungsgases in Form von Erwärmung und Befeuchtung auf natürliche Weise erhalten bleibt, gelangt bei invasiver Beatmung das Beatmungsgas nicht über den Nasen- und Rachenraum des Patienten und daher fällt ein großer Teil der bei natürlicher Atmung erfolgenden Konditionierung des Beatmungsgases weg. Bei der aktiven Beatmung wird das Beatmungsgas an den Patienten mit einer bestimmten Durchflussrate, auch Flowrate genannt, geliefert, die von der jeweiligen Beatmungssituation abhängt. Die Beatmungsgaskonditionierung ist auch bei normaler Beatmung häufig notwendig, weil das in Krankenhäusern üblicherweise aus einer zentralisierten Beatmungsgasquelle gelieferte Beatmungsgas so kalt und trocken ist, dass es von den oberen Atemwegen allein nicht ausreichend konditioniert werden kann.

Daher ist eine externe Konditionierung des bei der aktiven Beatmung verabreichten Beatmungsgases, d.h. eine aktive Erwärmung und Sättigung des Beatmungsgases mit Wasserdampf vor dessen Verabreichung an den Patienten, weit verbreitet, was zusätzliche gesundheitliche Risiken des Patienten begrenzt und als klinische Methode anerkannt ist.

Bei nicht invasiver Beatmung über eine Nasen- und/oder Gesichtsmaske wird in der Regel eine druckgesteuerte oder druckunterstützte Beatmungsform gewählt. Diese Form der Beatmung zeichnet sich durch eine dezelerierende Flowrate aus, die durch einen zu Beginn hohen und dann rasch abnehmenden Durchflusswert gekennzeichnet ist. Dies führt zu einer wirksamen Beatmung bei Verteilungsstörungen in der Lunge und zur Senkung des Beatmungsspitzendruckes. Die druckgesteuerte oder druckunterstützte Beatmungsform kompensiert sehr gut Leckagen z.B. bei Masken oder ungeblockten Trachealkanülen. Solche Leckagen werden dann vom Beatmungsgerät durch Erhöhung der Flowrate kompensiert. Die für die Beatmungsgaskonditionierung verwendete Beatmungsgaskonditionierungseinrichtung, auch Adapter genannt, soll auch bei hohen Flowraten eine ausreichende Anfeuchtung des Atemgases gewährleisten. Von hohen Flowraten, auch Hi-Flow genannt, spricht man bei Durchflussmengen von 60 Liter/min. und mehr.

Bisher lässt sich insbesondere bei nicht-invasiver Beatmung eine adäquate Konditionierung in der Konditionierungskammer bei hohen Flowraten (Hi-Flow) nicht erreichen.

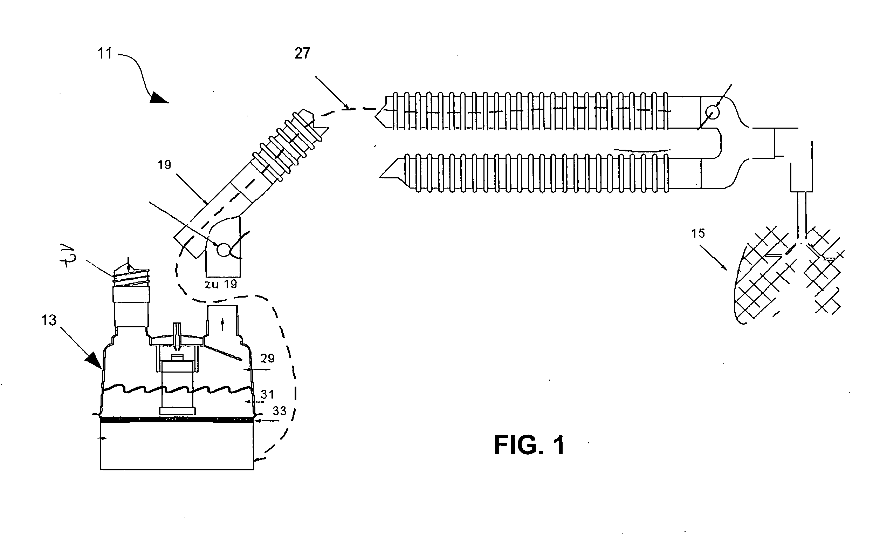

Figur 1 zeigt in schematischer Darstellung eine Beatmungseinrichtung 11 zur aktiven Beatmung mit einer Beatmungsgaskonditionierungseinrichtung 13 zur Konditionierung des der Lunge 15 eines Patienten zugeführten Beatmungsgases. Dieses stammt von einem nicht gezeigten Auslassanschluss einer nicht gezeigten Beatmungsgasquelle, z.B. von einer zentralen Beatmungsgasversorgung eines Krankenhauses. Zum Schutz des Leitungssystems dieser zentralen Beatmungsgasversorgung liefert diese üblicherweise trockenes Beatmungsgas und zwar etwa bei Zimmertemperatur. Daher muss das vom Auslassanschluss abgenommene Beatmungsgas mittels der Beatmungsgaskonditionierungseinrichtung 13 konditioniert, d.h. auf eine derartige Temperatur und einen derartigen Feuchtigkeitsgehalt gebracht werden, dass das Beatmungsgas in der Lunge 15 des Patienten mit richtiger Temperatur und Feuchtigkeit ankommt, welche den bei natürlicher Atmung mit Hilfe des Nasen-Rachen-Raums erhaltenen Werten in etwa entsprechen. Die Beatmungsgaskonditionierungseinrichtung 13 erhält eingangsseitig über einen Verbindungsschlauch 17 das von der Beatmungsgasquelle gelieferte Beatmungsgas, konditioniert das angelieferte Beatmungsgases hinsichtlich Temperatur und Feuchtigkeit und liefert das konditionierte Beatmungsgas über einen ausgangsseitig angeschlossenen Beatmungsschlauches 19, der zur Vermeidung des Entstehens von Kondensatflüssigkeit mit einem Heizdraht 27 versehen ist, an den Patienten. Soll das an den Patienten abgegebene Beatmungsgas beispielsweise eine Temperatur von 38°C aufweisen, wird am Ausgang der Beatmungsgaskonditionierungseinrichtung 13 Beatmungsgas mit einer Temperatur von beispielsweise 36°C abgegeben, unter der Annahme, dass in dem geheizten Beatmungsschlauch 19 eine zusätzliche Erwärmung um 2°C stattfindet.

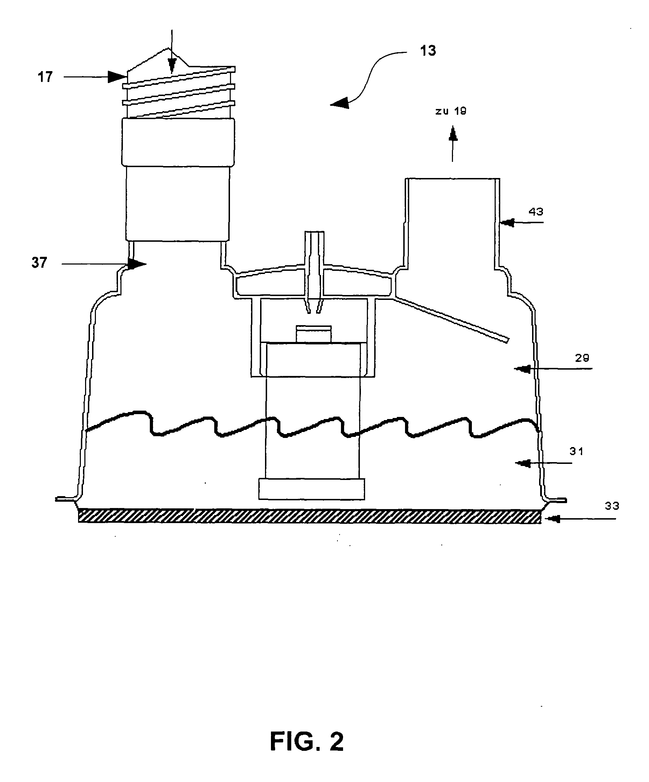

Eine für die Beatmungseinrichtung 11 bisher verwendete Beatmungsgaskonditionierungseinrichtung 13, wie sie in Figur 2 schematisch dargestellt ist, weist eine Konditionierungskammer 29 auf, die bei ordnungsgemäßem Betrieb eine vorbestimmte Menge einer Flüssigkeit, üblicherweise Wasser 31, enthält. Das Wasser 31 wird mit einer Heizplatte 33 elektrothermisch erwärmt. Das eingeleitete Beatmungsgas streicht über die Wasseroberfläche und wird dabei nach Maßgabe voreingestellter Parameter konditioniert und über den ausgangsseitigen Beatmungsschlauch 19 zum Patienten geführt. Bis zu einer bestimmten Durchflussrate des Beatmungsgases ist die Ausdehnung der Wasseroberfläche zur Sicherstellung der gewünschten Konditionierung des von der Konditionierungskammer 29 abgegebenen Beatmungsgases ausreichend. Wird ein dieser bestimmten Durchflussrate entsprechender Schwellenwert überschritten, wie im Falle des Hi-Flow, wird das Wasser 31 der Konditionierungskammer 29 so schnell abgekühlt, dass die Heizplatte 33 die erforderliche Temperatur zur Erzeugung des erforderlichen Wasserdampfs nicht mehr liefern kann und somit die gewünschte Beatmungsgaskonditionierung unmöglich ist. Außerdem geschieht mit zunehmender Durchflussrate ein Wärme- und Feuchtigkeitstausch zwischen dem Wasser 31 und dem über die Oberfläche des Wassers 31 geleiteten Beatmungsgas im Wesentlichen nur in einer relativ dünnen wasseroberflächennahen Schicht des Wassers, die mit zunehmender Durchflussrate des Beatmungsgases zum Temperaturausgleich mit dem darunter liegenden Wasservolumen immer weniger in der Lage ist und daher immer mehr abkühlt, sodass bei zunehmender Durchflussrate des Beatmungsgases auch durch eine Erhöhung der Heizleistung der Heizplatte 33 in vertretbarem Ausmaß eine ausreichende Erwärmung und Befeuchtung des Beatmungsgases nicht mehr sicher gestellt werden kann

Bei den bekannten Beatmungsanlagen üblicher Art werden Einweg-Konditionierungskammern mit einem bestimmten Aufbau und einer bestimmten Spezifikation eingesetzt, bei deren Beibehaltung die Heizleistung nicht erhöht und aus den genannten Gründen eine ausreichende Konditionierung bei Hi-Flow nicht mehr zufriedenstellend erreicht werden kann.

Es ist daher erwünscht, eine Konditionierungskammer verfügbar zu machen, die selbst dann, wenn sie im Wesentlichen den Aufbau und die Spezifikationen der bekannten Konditionierungskammer aufweist, eine ausreichende Konditionierung des bei aktiver Beatmung einem Patienten zugeführten Beatmungsgases auch bei Hi-Flow ermöglicht. Insbesondere soll es ermöglicht werden, dem zu beatmenden Patienten Beatmungsgas mit 37°C und einer relativen Feuchtigkeit von 100% entsprechend 44mg Feuchtigkeit/I Beatmungsgas auch bei Hi-Flow zuzuführen.

Die Erfindung schafft ein Sprudelungsrohr nach Patentanspruch 1, eine Beatmungskonditionierungseinrichtung nach Patentanspruch 8, insbesondere in Kombination mit einem erfindungsgemäßen Sprudelungsrohr, einen Verbindungsschlauch nach Anspruch 12, insbesondere für den Eisatz in einer erfindungsgemäßen Beatmungskonditionierungseinrichtung, und ein Verfahren zur Temperatur- und Feuchte-Konditionierung von einem Patienten zuzuführendem Beatmungsgas nach Patentanspruch 13. Ausbildungen hiervon sind in den abhängigen Patentansprüchen angegeben.

Wenn im Zusammenhang mit der vorliegenden Erfindung der Einfachheit halber meist von Wasser gesprochen wird, sollen andere Flüssigkeiten nicht ausgeschlossen sondern miteinbezogen sein.

Nach einem ersten Aspekt schafft die Erfindung eine Beatmungsgaskonditionierungseinrichtung. Diese weist eine Konditionierungskammer auf, welcher von einer Beatmungsgasquelle Beatmungsgas zugeführt wird, das in der Konditionierungskammer konditioniert, das heißt auf vorbestimmte Weise erwärmt und befeuchtet, und dann an einen zu beatmenden Patienten abgegeben wird. Die Konditionierungskammer ist zur Befüllung mit einer das Beatmungsgas befeuchtenden Flüssigkeit ausgebildet. Sie weist einen Kammereinlass auf, der zur Verbindung mit der Beatmungsgasquelle ausgebildet ist. Sie besitzt einen Kammerauslass, der zur Verbindung mit dem Patienten ausgebildet ist. In der Konditionierungskammer ist zur Erwärmung der Flüssigkeit eine Flüssigkeitsheizeinrichtung vorgesehen. Außerdem ist eine Sprudelungseinrichtung vorgesehen, die derart ausgebildet und in der Konditionierungskammer derart angeordnet ist, dass sie mindestens einen Teil des von der Beatmungsgasquelle kommenden Beatmungsgases in mindestens einen Teil des Volumens der in der Konditionierungskammer befindlichen Flüssigkeit zur Erwärmung und Befeuchtung des Beatmungsgases mithilfe dieses Flüssigkeitsvolumens hineinsprudelt.

Durch das Hineinsprudeln von Beatmungsgas in das Volumen der Flüssigkeit, bei der es sich üblicherweise um Wasser handelt, möglicherweise auch um Wasser, dem eine medizinisch wirksames Mittel zugesetzt ist, wird erfindungsgemäß zusätzlich zu der Austauschzone im Bereich der Wasseroberfläche, wie sie bei der bekannten Beatmungsgaskonditionierungseinrichtung ausschließlich wirksam ist, eine hochwirksame zusätzliche Austauschzone für den das Beatmungsgas konditionierenden Austausch zwischen Flüssigkeit und Beatmungsgas innerhalb mindestens eines großen Teils des Flüssigkeitsvolumens geschaffen. Dies führt zu einer Vergrößerung der Austauschfläche zwischen Beatmungsgas und Flüssigkeit und damit zu einer beträchtlichen Erhöhung der Austauscheffizienz zwischen Beatmungsgas und Flüssigkeit, also zu einer entsprechenden Erhöhung des Wärme- und Feuchtigkeitsaustauschs zwischen Flüssigkeit und Beatmungsgas. Daher kann auch bei Hi-Flow eine ausreichende Konditionierung des Beatmungsgases gewährleistet werden, selbst wenn die bei der bekannten Beatmungsgaskonditionierungseinrichtung zum Einsatz kommende Heizplattenheizleistung unverändert bleibt. Außerdem wird die Strömungsform verbessert und eine Schichtung von Flüssigkeit und Beatmungsgas über einander vermieden.

Durch die erfindungsgemäße Sprudelungseinrichtung wird also erreicht, dass anders als bei der bekannten Konditionierungskammer eine Aufladung des Beatmungsgases mit Wärme und Feuchtigkeit nicht nur in einem relativ kleinen Bereich des Wasservolumens im Bereich von dessen Wasseroberfläche stattfindet sondern durch die Einsprudelung des Beatmungsgases unter die Wasseroberfläche ein wesentlich größerer Bereich des Wasservolumens an der Aufladung des Beatmungsgases mit Wärme und Feuchtigkeit beteiligt wird.

Bei einer Ausführungsform der Erfindung ist die Flüssigkeitsheizeinrichtung in an sich bekannter Weise als eine Heizplatte ausgebildet, die bei ordnungsgemäßer Flüssigkeitsbefüllung der Konditionierungskammer in die Flüssigkeit eingetaucht ist.

Bei einer Ausführungsform der Erfindung bildet die Heizplatte für die Flüssigkeit eine Bodenplatte.

Bei einer Ausführungsform der Erfindung ist die Sprudelungseinrichtung heizbar. Damit wird eine wirksamere Wärmekonditionierung und damit auch höhere Feuchtigkeitsaufnahme des Beatmungsgases erreicht.

Bei einer Ausführungsform der Erfindung ist die Sprudelungseinrichtung mittels einer zur Flüssigkeitsheizeinrichtung zusätzlich vorgesehenen Zusatzheizeinrichtung heizbar.

Bei einer Ausführungsform der Erfindung ist Sprudelungseinrichtung durch Kontakt mit der Flüssigkeitsheizeinrichtung heizbar. Eine Zusatzheizeinrichtung kann daher entfallen und die wirksamere Wärmekonditionierung und höhere Feuchtigkeitsaufnahme können trotzdem erreicht werden.

Bei einer Ausführungsform der Erfindung ist die Sprudelungseinrichtung als Sprudelungsrohr, welches als Diffusionsrohr wirkt, ausgebildet, das auf einer Einlassseite mit dem Kammereinlass verbunden ist und eine Auslassseite aufweist, die bei ordnungsgemäßer Flüssigkeitsbefüllung der Konditionierungskammer mindestens teilweise in die Flüssigkeit eingetaucht ist.

Bei einer Ausführungsform der Erfindung besteht das Sprudelungsrohr aus Metall, hat somit eine gute Wärmeleitung, so dass es sich durch Berührung der Heizplatte der Konditionierungskammer über mindestens einen großen Teil seiner Länge im wesentlichen gleichmäßig erwärmen lässt.
Insbesondere wenn eine Erwärmung des Sprudelungsrohrs durch den geheizten Kammerboden für nicht erforderlich gehalten wird, braucht das Sprudelungsrohr den Kammerboden nicht zu berühren und braucht das Sprudelungsrohr auch nicht aus Metall zu sein.

Bei einer Ausführungsform der Erfindung ist das Sprudelungsrohr im Bereich eines Beatmungsgas-Einlasses der Konditionierungskammer angeordnet, so dass das gesamte Wasservolumen von der Einlassseite bis zur Auslassseite der Konditionierungskammer für die Aufladung des Beatmungsgases mit Feuchtigkeit und Wärme genutzt werden kann.

Um einen möglichst großen Anteil des Flüssigkeitsvolumens mit dem Beatmungsgas zu versprudeln, wird das Sprudelungsrohr auch dann, wenn es mit der Flüssigkeitsheizeinrichtung nicht in Kontakt gebracht wird, mit seinem versprudelnd wirkenden Rohrbereich möglichst tief in die Flüssigkeit eingetaucht.

Bei einer Ausführungsform der Erfindung ist das Sprudelungsrohr an seinem in die Flüssigkeit getauchten Ende offen und bildet dieses offene Rohrende eine einzige Diffusionsöffnung.

Bei einer Ausführungsform der Erfindung weist das Sprudelungsrohr an seiner Auslassseite eine Mehrzahl von Diffusionsöffnungen auf. Diese bewirken eine Sprudelungswirkung in einem entsprechend großen Bereich des das Sprudelungsrohr umgebenden Flüssigkeitsvolumens und damit die Ausnutzung eines großen Teils des Flüssigkeitsvolumens zur Konditionierung des Beatmungsgases.

Bei einer Ausführungsform der Erfindung ist das Sprudelungsrohr an einem auslassseitigen Rohrende geschlossen und sind die Diffusionsöffnungen um dessen Rohrumfang verteilt. Damit wird eine größere Gleichmäßigkeit der Sprudelung als bei einem lediglich am unteren Ende offenen Sprudelungsrohr erzielt.

Bei einer Ausführungsform der Erfindung sind in unterschiedlicher axialer Position des Sprudelungsrohrs je eine Mehrzahl von um dessen Rohrumfang verteilte Diffusionsöffnungen vorgesehen. Dies dient der Einbeziehung eines größeren Teils der Flüssigkeitsäule des Flüssigkeitsvolumens in eine möglichst gleichförmige Versprudelung.

Bei einer Ausführungsform der Erfindung wird ein Sprudelungsrohr in Form eines geraden Rohrstücks verwendet, das sich im wesentlichen senkrecht zur Oberfläche des in der Konditionierungskammer befindlichen Wassers erstreckt.

Bei einer Ausführungsform der Erfindung wird ein Sprudelungsrohr verwendet, welches in einem bei ordnungsgemäßer Wasserbefüllung der Konditionierungskammer in das Wasser eingetauchten Rohrbereich abgebogen ist, so dass es ein vorzugsweise parallel zum Boden der Konditionierungskammer verlaufendes Rohrenstück aufweist, in welchem die Diffusionsöffnungen angeordnet sind. Mit einem derartig abgebogenen Sprudelungsrohr lässt sich erreichen, dass eine Sprudelung über einen größeren Erstreckungsbereich des Wasservolumens stattfindet als bei einem geraden Rohrstück.

Während bei Wasserdampf das Risiko der Keimübertragung minimiert ist, kann Wasser Keime und Viren transportieren. Daher ist darauf zu achten, dass die durch die Sprudelung verursachte Strömung im Wasser nicht zu stark wird und es nicht zu einer so starken Aufsprudelung an der Wasseroberfläche kommt, dass Wassertropfen in den Beatmungsschlauch und mit diesen Keime und Viren zum Patienten gelangen können.

Um eine übermäßige Sprudelung an der Wasseroberfläche zu vermeiden, weist bei einer Ausführungsform der Erfindung das Sprudelungsrohr zusätzlich zu den Diffusionsöffnungen mindestens eine weitere Auslassöffnung auf, die bei ordnungsgemäßer Flüssigkeitsbefüllung der Konditionierungskammer nicht in die Flüssigkeit eingetaucht ist und dazu dient, die Sprudelwirkung an der Flüssigkeitsoberfläche abzubremsen. Damit wird eine Sprudelung an der Flüssigkeitsoberfläche derart, dass eine Hineinsprudelung von Flüssigkeit in den Beatmungsschlauch auftreten könnte, vermieden.

Bei einer Ausführungsform der Erfindung ist eine Mehrzahl von Auslassöffnungen gleichmäßig um den Umfang des Sprudelungsrohrs angeordnet, um eine möglichst allseitige Abbremsung oder Beruhigung der Sprudelungswirkung an der Wasseroberfläche zu bewirken.

Bei einer Ausführungsform der Erfindung sind die eine oder die mehreren Auslassöffnungen größer dimensioniert als die Diffusionsöffnungen. Damit wird erreicht, dass die Sprudelungsverminderung oder -vermeidung an der Flüssigkeitsoberfläche stärker ist als die Sprudelungswirkung im Flüssigkeitsvolumen.

Bei einer Ausführungsform der Erfindung ist der axiale Bereich, in welchem die mindestens eine Auslassöffnung angeordnet ist, als sich in Richtung des auslassseitigen Rohrendes verjüngender Abschnitt des Sprudelungsrohrs ausgebildet.

Die Diffusionsöffnungen und die weiteren Auslassöffnungen können teilweise oder insgesamt rund oder rechteckig ausgebildet sein.

Bei einer Ausführungsform der Erfindung werden die Diffusionsöffnungen in einem Rohrendbereich des Sprudelungsrohrs durch die Rohrwand durchsetzende Wandöffnungen gebildet. Bei einer anderen Ausführungsform wird der für die Besprudelung des Wassers verwendete Rohrendbereich durch eine in Hohlzylinderform gebrachte, beispielsweise netz- oder gitterförmige Maschenware, gebildet,
wobei die Zylinderform der Maschenware an die Zylinderform des Sprudelungsrohrs angepasst ist.

Eine erfindungsgemäße Beatmungsgaskonditionierungseinrichtung kann einen gänzlich anderen Aufbau haben als die herkömmlich verwendete Beatmungsgaskonditionierungseinrichtung. Ein besonderer Vorteil der Erfindung besteht aber darin, dass eine herkömmliche Beatmungsgaskonditionierungseinrichtung leicht zu einer erfindungsgemäßen Beatmungsgaskonditionierungseinrichtung aufgerüstet werden kann. Zu diesem Zweck braucht die herkömmliche Konditionierungskammer lediglich nachträglich mit der erfindungsgemäßen Sprudelungseinrichtung versehen zu werden. Bei Ausbildung der Sprudelungseinrichtung als Sprudelungsrohr kann dieses nach Art eines Adapters im Bereich des Kammereinlasses in die herkömmliche Konditionierungskammer eingesetzt werden. Dabei kann das Sprudelungsrohr vor seiner Verbindung mit dem Verbindungsschlauch in die Konditionierungskammer eingesetzt werden oder kann der mit der Eingangsseite der Konditionierungskammer verbundene Bereich des die Konditionierungskammer mit der Beatmungsgasquelle koppelnden Verbindungsschlauchs mit dem erfindungsgemäßen Sprudelungsrohr bestückt werden, bevor der Verbindungsschlauch mit der Konditionierungskammer verbunden wird.

Bei einer Ausführungsform der Erfindung, bei welcher das Sprudelungsrohr als mit Diffusionsöffnungen versehenes Metallrohr ausgebildet ist, das bis zum erwärmten Kammerboden reicht, stellen sich folgende Effekte ein: Einerseits wird das Metallrohr durch Berührung des erwärmten Kammerbodens gleichmäßig erwärmt und gibt das Metallrohr diese Wärme an das Beatmungsgas und an das Wasser ab. Andererseits wird anhand der Diffusionsöffnungen in dem Metallrohr unter der Wasseroberfläche eine Strömung erzeugt, was die Austauscheffizienz zwischen Atemgas und Wasser erhöht. Auf diese Weise kann die angestrebte Feuchtigkeit des Beatmungsgases bei nicht-invasiver Beatmung auch bei Hi-Flow erreicht werden. Das Beatmungsgas wird dem Patienten mit bedarfsabhängigen Strömungsmengen verabreicht. Angesichts dessen ist bei einer Ausführungsform der Erfindung der durch das Sprudelungsrohr gebildete Adapter steuerbar ausgebildet, derart, dass die Menge des an den Patienten gelieferten Beatmungsgases zur Anpassung an den Beatmungsbedarf verstellbar ist.

Bei einer Ausführungsform der Erfindung ist das Sprudelungsrohr mit einer Drosselvorrichtung versehen, mittels welcher die Menge des in die Flüssigkeit sprudelnden Beatmungsgases gesteuert werden kann.

Bei einer Ausführung der Erfindung handelt es sich bei der Drosselvorrichtung um eine Blende in Form eines auf die Außenseite des Sprudelungsrohr aufgesetzten außenseitigen Aufsatzes und/oder in Form eines auf der Innenseite des Sprudelungsrohrs befindlichen innenseitigen Einsatzes, die relativ zu dem mit der mindestens einen Diffusionsöffnung versehenen Teil des Sprudelungsrohrs derart bewegbar ist, dass ein wählbarer Anteil der mindestens einen Diffusionsöffnung des Sprudelungsrohrs verschlossen werden kann.

Nach einem zweiten Aspekt schafft die Erfindung ein Sprudelungsrohr, welches für eine Beatmungsgaskonditionierungseinrichtung verwendbar ist und mindestens eines der Merkmale aufweist, welche oben im Zusammenhang mit dem ersten Aspekt für eine in einer Konditionierungskammer angeordnete Sprudelungseinrichtung angegeben worden sind.

Nach einem dritten Aspekt schafft die Erfindung einen Verbindungsschlauch, ausgebildet für die Verbindung einer Einlassseite einer Konditionierungskammer einer Beatmungsgaskonditionierungseinrichtung mit einer Beatmungsgasquelle, wobei ein für die Verbindung mit der Einlassseite der Konditionierungskammer ausgebildeter Endbereich des Verbindungsschlauchs mit einem Sprudelungsrohr mit mindestens einem der im Zusammenhang mit dem zweiten Aspekt angegebenen Merkmale versehen ist.
Nach einem vierten Aspekt schafft die Erfindung ein Verfahren zur Temperatur- und Feuchte-Konditionierung von einem Patienten zuzuführendem Beatmungsgas durch Wärme- und Feuchtigkeitsaustausch mit einer in eine Konditionierungskammer gefüllten Flüssigkeit, wobei das Beatmungsgas mittels einer Sprudelungseinrichtung in einen unterhalb der Flüssigkeitsoberfläche liegenden Volumenbereich der Flüssigkeit eingesprudelt wird. Dazu kann ein Sprudelungsrohr mit mindestens einem der Merkmale gemäß dem zweiten Aspekt verwendet werden. dieses Verfahren kann bei einer Beatmungsgaskonditionierungseinrichtung mit mindestens einem der Merkmale nach dem ersten Aspekt der Erfindung durchgeführt werden.

Als Alternativen zu der erfindungsgemäßen Sprudelungseinrichtung für eine Verbesserung der Austauscheffizienz zwischen Beatmungsgas und Wasser sind einerseits eine größere Konditionierungskammer und andererseits eine auf der Außenseite der Befeuchtungskammer angeordnete Vorrichtung zur Erzeugung einer Wasserströmung in der Befeuchterkammer denkbar. Solche Alternativen haben aber einen entscheidenden Nachteil. Da es sich bei den marktüblichen Konditionierungskammern in der Regel um quasi-genormte Einweg-Produkte handelt und diese auch an verschiedenen Konditionierungskammern betreibbar sein sollen, wären die genannten Alternativen mangels ausreichender Kompatibilität mit den verschiedenen marktüblichen Atemgaskonditionierungseinrichtungen und/oder aus Kostengründen nicht marktfähig.

Die Erfindung wird nun anhand der beigefügten Zeichnungen noch zusätzlich erläutert. In den Zeichnungen zeigen:
- Figur 1: eine Beatmungseinrichtung der bereits erwähnten Art;
- Figur 2: eine bei der Beatmungseinrichtung nach Figur 1 verwendbare bekannte Beatmungsgaskonditioniereinrichtung wie ebenfalls bereits erwähnt;
- Figur 3: eine bei der Beatmungseinrichtung nach Figur 1 verwendbare erste Ausführungsform einer erfindungsgemäßen Beatmungsgaskonditioniereinrichtung;
- Figur 4: eine Ausführungsform eines bei der Beatmungsgaskonditioniereinrichtung gemäß Figur 3 verwendbaren erfindungsgemäßen Sprudelungsrohrs;
- Figur 5: eine bei der Beatmungseinrichtung nach Figur 1 verwendbare zweite Ausführungsform einer erfindungsgemäßen Beatmungsgaskonditioniereinrichtung;
- Figur 6: eine bei der Beatmungseinrichtung nach Figur 1 verwendbare dritte Ausführungsform einer erfindungsgemäßen Beatmungsgaskonditioniereinrichtung mit Drosselvorrichtung; und
- Figur 7: eine Ausführungsform eines bei der Beatmungsgaskonditioniereinrichtung gemäß Figur 6 verwendbaren erfindungsgemäßen Sprudelungsrohrs mit Drosselvorrrichtung.

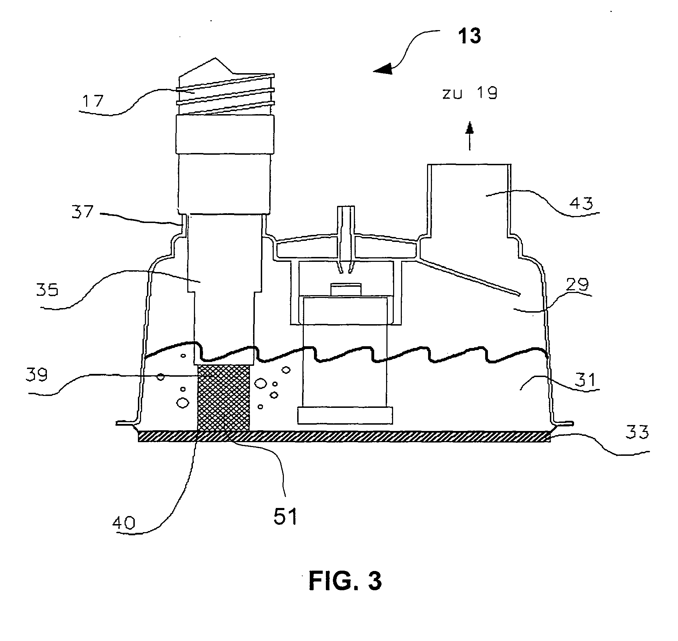

Die in Figur 3 gezeigte Ausführungsform einer erfindungsgemäßen Beatmungsgaskonditioniereinrichtung 13 weist weitgehend den gleichen Aufbau auf wie die in Figur 2 gezeigte bekannte Beatmungsgaskonditioniereinrichtung, weswegen für gleiche Komponenten auch die gleichen Bezugszeichen wie in Figur 2 verwendet werden, ist jedoch erfindungsgemäß zusätzlich mit einer Sprudelungseinrichtung versehen, die bei dieser Ausführungsform der Erfindung als Sprudelungsrohr 35 in Form eines Eintauchrohres ausgebildet ist, das als Diffusionsrohr wirkt.

Eine herkömmliche Konditionierungskammer kann dadurch zu einer erfindungsgemäßen Konditionierungskammer 29 gemacht werden, dass sie nachträglich mit dem Sprudelungsrohr 35 versehen wird. Zu diesem Zweck wird der eingangsseitige Verbindungsschlauch 17 im Bereich einer zu dessen Anschluss vorgesehenen Einlassstütze 37 der Konditionierungskammer 29 an das Sprudelungsrohr 35 angeschlossen, welches über die Einlassstütze 37 so weit in die Konditionierungskammer 29 eingeführt ist, dass sein von der Einlassstütze 37 abliegendes Rohrendbereich 38 in das Wasser 31 eingetaucht ist. Dabei kann das Sprudelungsrohr 35 entweder über die Einlassstütze 37 in die Konditionierungskammer 29 eingesetzt werden, woraufhin dann der Verbindungsschlauch 17 mit dem Sprudelungsrohr 35 verbunden wird, oder der Verbindungsschlauch 17 wird mit dem Sprudelungsrohr 35 versehen, bevor dieser mit der Konditionierungskammer 29 verbunden wird. Im Letzteren Fall kann der Verbindungsschlauch 17 beispielsweise bereits bei seiner Herstellung oder vor seiner Auslieferung mit dem Sprudelungsrohr 35 konfektioniert werden.

Bei der in Figur 3 dargestellten ersten Ausführungsform der Erfindung ist das Sprudelungsrohr 35 mit als Diffusionsöffnungen 39 wirkenden Auslassöffnungen versehen, die bei einer ordnungsgemäßen Wasserfüllung der Konditionierungskammer 29 alle unterhalb der Wasseroberfläche liegen. Bei der dargestellten Ausführungsform werden die Diffusionsöffnungen 39 durch die Maschenöffnungen einer im wesentlichen hohlzylindrisch angeordneten Maschenware 51 gebildet. Im Fall eines Sprudelungsrohrs 35, dessen Querschnitt anders als kreisförmig ist, wird die Maschenware 51 in entsprechender Form angeordnet. bei einer Ausführungsform der Erfindung handelt es sich bei der Maschenware 51 um ein Netz oder Gitter, beispielsweise aus metallischem Material oder Kunststoff. Beispielsweise kann ein Gitter verwendet werden, wie es in Wasserhähnen unter der Bezeichnung "Perlator" zum Einsatz kommt. Für den erfindungsgemäßen Zweck eignet sich als Maschenware 51 auch Filtermaterial, das als Blutfilter Verwendung findet. Mit auf diese Weise geschaffenen Diffusionsöffnungen wird eine gleichmäßige Versprudelung des zugeführten Beatmungsgases in dem das Sprudelungsrohr 35 umgebenden Wasser 31 und damit eine Beteiligung eines großen Teils des Wasservolumens an der Konditionierung des Beatmungsgases erreicht. Die Diffusionsöffnungen 39 können aber auch auf andere Weise und in anderer Anordnung am Sprudelungsrohr 35 vorgesehen sein.

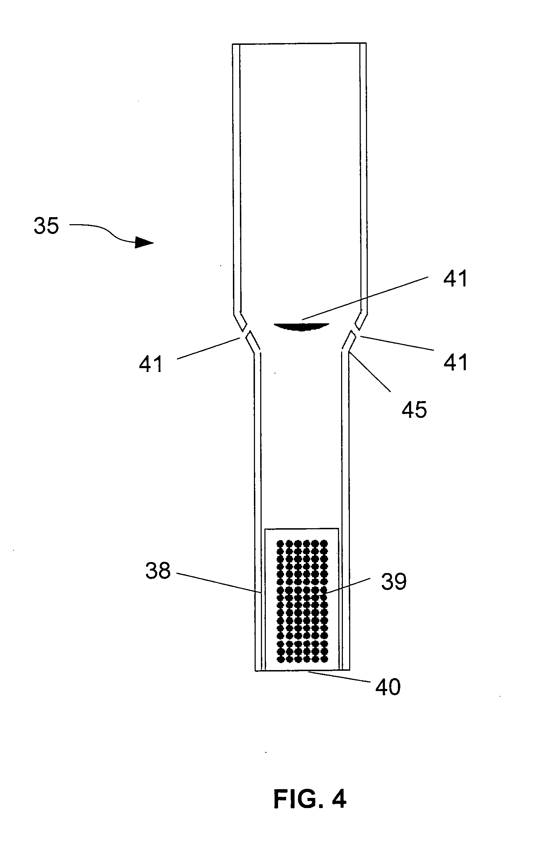

Bei einer in Figur 4 dargestellten Ausführungsform eines erfindungsgemäßen Sprudelungsrohrs 35 ist der Rohrendbereich 38 als Rohrstück ausgebildet, bei welcher die Durchgangsöffnungen 39 als Löcher in der Rohrwand ausgebildet sind. Bei der dargestellten Ausführungsform ist eine Vielzahl Diffusionsöffnungen 39 vorgesehen, die in mehreren in Axialrichtung des Sprudelungsrohrs 35 übereinander liegenden Ebenen vorzugsweise gleichmäßig um den Umfang des Sprudelungsrohrs 35 verteilt sind. Auf diese Weise wird auch bei dieser Ausführungsform eine gleichmäßige Versprudelung des zugeführten Beatmungsgases in dem das Sprudelungsrohr 35 umgebenden Wasser 31 und damit eine Beteiligung eines großen Teils des Wasservolumens an der Konditionierung des Beatmungsgases erreicht. Die Diffusionsöffnungen 39 können aber auch in anderer Anordnung am Sprudelungsrohr 35 vorgesehen sein. Beispielsweise können die Diffusionsöffnungen 39 wendelförmig um einen unteren Bereich des Sprudelungsrohrs 35 angeordnet sein oder auch unregelmäßig.

Bei der in Figur 4 dargestellten Ausführungsform haben sämtliche Diffusionsöffnungen 39 eine in etwa kreisrunde Form. Sie können jedoch auch eine andere Form haben, beispielsweise rechteckig, quadratisch, oval, elliptisch, dreieckig, rautenförmig usw. seien.

Bei der in Figur 4 gezeigten Ausführungsform ist das Sprudelungsrohr 35 in einem axialen Bereich, der bei ordnungsgemäßer Wasserfüllung der Konditionierungskammer 29 oberhalb der Wasseroberfläche liegt, mit einer oder mehreren weiteren Auslassöffnungen 41 versehen, welche dazu dienen, die erzeugte Sprudelwirkung im Bereich der Wasseroberfläche abzubremsen. Damit wird verhindert, dass an der Wasseroberfläche eine Sprudelung in solchem Maß entsteht, dass sprudelndes Wasser in den an eine Auslassstütze 43 der Konditionierungskammer 29 angeschlossenen Beatmungsschlauches 19 hinein sprudelt. Bei der in Figur 4 gezeigten Ausführungsform eines erfindungsgemäßen Sprudelungsrohrs 35 sind diese weiteren Auslassöffnungen 41 alle in der gleichen axialen Ebene des Sprudelungsrohrs 35 angeordnet. Auch hinsichtlich dieser weiteren Auslassöffnungen 41 sind jedoch andere Anordnungen möglich. Außerdem haben bei der in Figur 4 dargestellten Ausführungsform die mehreren dort dargestellten weiteren Auslassöffnungen 41 je die Form eines Kreissegments. Auch hierfür sind nahezu beliebig anderer Öffnungsformen möglich. Bei der dargestellten Ausführungsform befinden sich die weiteren Auslassöffnungen 41 in einem sich nach unten zu verjüngenden axialen Abschnitt 45 des Sprudelungsrohrs 35.

Damit wird erreicht, dass die aus den weiteren Auslassöffnungen 45 austretenden Beatmungsgasströme schräg auf die Wasseroberfläche des in der Konditionierungskammer 29 befindlichen Wassers geblasen werden. Damit wird eine besonders wirksame Beruhigung der Wasseroberfläche erzielt.

Da sich die bekannte Konditionierungskammer durch Bestückung mit dem Sprudelungsrohr 35 zu einer erfindungsgemäßen Konditionierungskammer 29 aufrüsten lässt, das Sprudelungsrohr 35 also zur Anpassung der bekannten Konditionierungskammer an eine erfindungsgemäße Konditionierungskammer 29 verwendet werden kann, wird das Sprudelungsrohr 35 auch als Adapter bezeichnet. Je nach Ausführungsform handelt es sich um einen steuerbaren oder nicht steuerbaren Adapter. In den Figuren 3 und 4 ist ein nicht steuerbarer Adapter gezeigt.

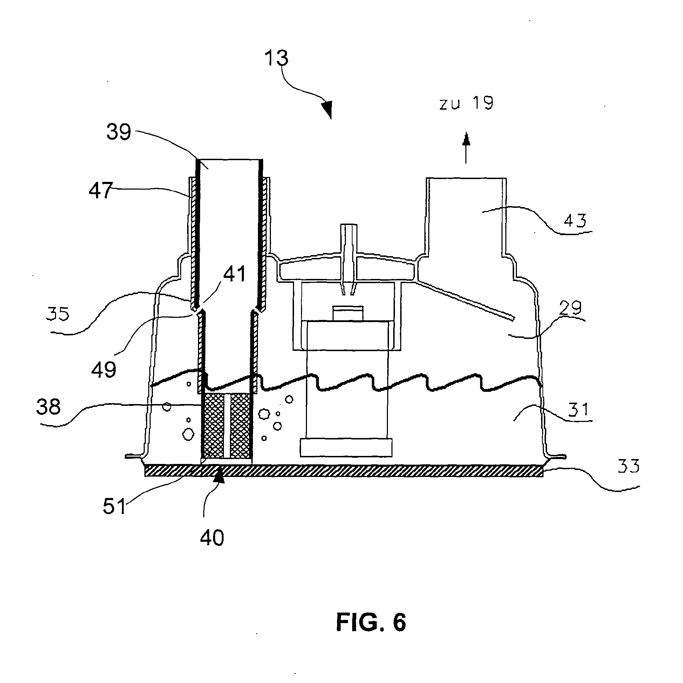

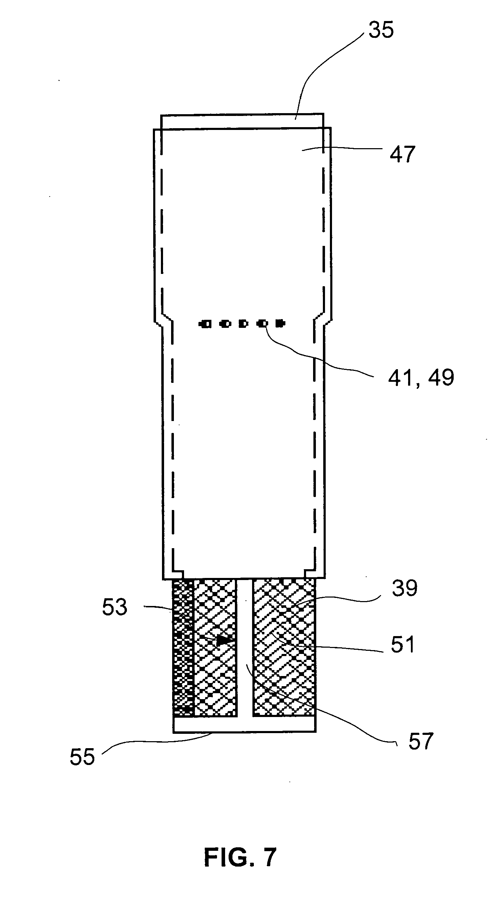

Eine in den Figuren 6 und 7 gezeigte und weiter unten näher erläuterte Ausführungsform der Erfindung weist einen steuerbaren Adapter auf, bei welchem das Sprudelungsrohr 35 in der bereits erwähnten Weise mit einem axial und/oder umfangsmäßig relativ zum Sprudelungsrohr bewegbaren Einsatz und/oder Aufsatz mit Drossel- oder Blendenfunktion versehen ist.

Bei der in Figur 3 dargestellten Ausführungsform der Erfindung ist das Sprudelungsrohr 35 so weit in das Wasser 31 eingetaucht, dass dessen Rohrende 40 mit der Heizplatte 33 in Kontakt kommt und das Sprudelungsrohr 35 auf diese Weise von der Heizplatte 33 aufgewärmt wird. Durch den Kontakt des Sprudelungsrohrs 35 mit der am Konditionierungskammerboden befindlichen Heizplatte 33 wird das Sprudelungsrohr 35 über seine Gesamtlänge erwärmt und bewirkt eine Vorwärmung des Beatmungsgases. Es besteht aber auch die Möglichkeit, eine Aufwärmung des Sprudelungsrohrs 35 zusätzlich oder lediglich über eine dem Sprudelungsrohr 35 separat zugeordnete, in der Zeichnung nicht dargestellte Zusatzheizvorrichtung zu bewirken. Das Beatmungsgas sprudelt unter der Wasseroberfläche aus dem Sprudelungsrohr 35 und wird bis zum Zielwert temperiert und mit Wasserdampf gesättigt. Das konditionierte Beatmungsgas wird über eine auf der Auslassseite der Konditionierungskammer 29 angeordnete Ausleitungsstütze 43 zum Patienten geführt.

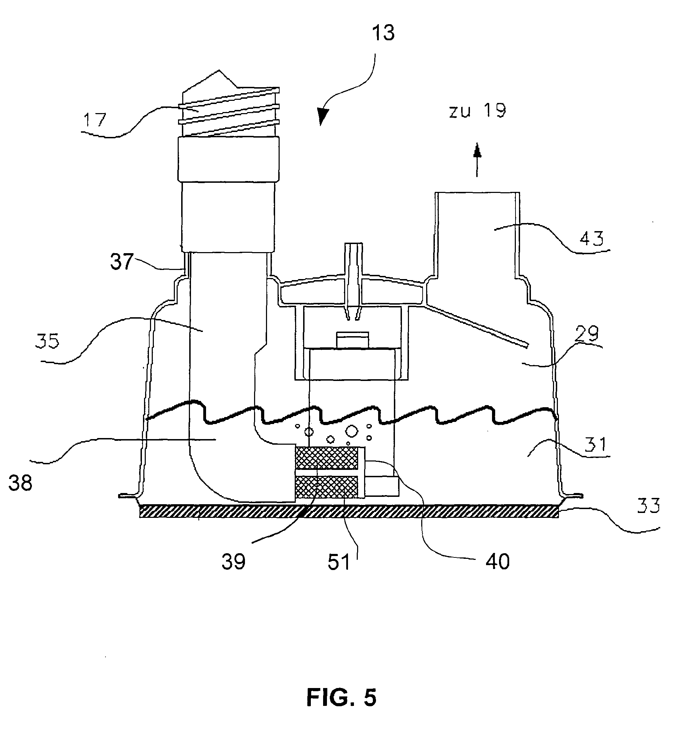

Figur 5 zeigt eine zweite Ausführungsform einer erfindungsgemäßen Beatmungsgaskonditioniereinrichtung 13, welche weitgehend mit der in Figur 3 gezeigten ersten Ausführungsform übereinstimmt, jedoch hinsichtlich der Ausbildung des Rohrendbereichs 38 von der Ausführungsform gemäß Figur 3 abweicht. Bei der Ausführungsform gemäß Figur 5 ist das Sprudelungsrohr 35 nicht als lineares Rohr ausgebildet sondern ist in seinem Rohrendbereich 38, der sich bei ordnungsgemäßer Füllung der Konditionierungskammer 29 mit Wasser 31 unterhalb der Wasseroberfläche befindet, derart abgebogen, dass sich der mit den Diffusionsöffnungen 39 versehene Teil des Sprudelungsrohr 35 mindestens in etwa parallel zur Heizplatte 33 erstreckt. Mit einer solchen Anordnung mit abgebogenem Rohrendbereich 38 kann dessen mit Diffusionsöffnungen 39 versehener Teil nach Bedarf wesentlich länger gemacht werden als bei der Verwendung eines linearen Sprudelungsrohrs 35 gemäß Figur 3. Dementsprechend kann bei der Ausführungsform nach Figur 5 in Richtung parallel zur Heizplatte 33 gesehen ein wesentlich größeres Wasservolumen besprudelt werden als bei Verwendung eines linearen Sprudelungsrohrs 35. Somit kann bei der Anordnung gemäß Figur 5 der Feuchtigkeits-und Temperaturaustausch zwischen dem Wasser 31 und dem in die Konditionierungskammer 29 eingeleiteten Beatmungsgas bei Bedarf größer gemacht werden als bei der in Figur 3 gezeigten Ausführungsform, wenn alle anderen Parameter unverändert bleiben, insbesondere die Diffusionsöffnungsstruktur sowie die Durchflussrate des Beatmungsgases.

Wenn eine Aufheizung des Sprudelungsrohrs 35 mit Heizenergie der Heizplatte 33 erwünscht ist, wird der parallel zur Heizplatte 33 verlaufende Teil des Rohrendbereichs 38 mit der Heizplatte 33 in Berührung gebracht.

Figur 6 zeigt eine dritte Ausführungsform einer erfindungsgemäßen Beatmungsgaskonditioniereinrichtung 13, welche ebenfalls weitgehend mit der in Figur 3 gezeigten ersten Ausführungsform übereinstimmt, jedoch mit einer Drosselungsvorrichtung versehen ist, mit welcher die Menge des aus dem Rohrendbereich 38 austretenden Beatmungsgases verändert werden kann, im Sinn eines zuvor erwähnten steuerbaren Adapters. Bei der in Figur 6 gezeigten Ausführungsform wird die Drosselungsvorrichtung durch ein Drosselrohr 47 gebildet, welches in der in Figur 6 gezeigten Weise auf die Außenwand des Sprudelungsrohrs 35 derart aufgesetzt ist, dass das Drosselrohr 47 relativ zum Sprudelungsrohr 35 axial verschiebbar und/oder um das Drosselrohr 47 verdrehbar ist. Beispielsweise kann man bei der in Figur 6 gezeigten Ausführungsform die Menge des durch die Diffusionsöffnungen 39 austretenden Beatmungsgases dadurch reduzieren oder gar auf null bringen, dass das Drosselrohr 47 über einen wählbaren Teil des mit den Diffusionsöffnungen 39 versehenen Rohrendbereichs 38 beziehungsweise bis zum Rohrende 40 des Sprudelungsrohrs 35 herab geschoben wird.

Wie Figur 6 zeigt, ist die Innenkontur des Drosselrohrs 47 an die Außenkontur des Sprudelungsrohrs 35 angepasst, weist also zwischen seinen Enden einen sich verjüngenden Bereich 48 auf, welcher an den sich verjüngenden Bereich 49 des Sprudelungsrohrs 35 angepasst ist. Bei der dargestellten Ausführungsform ist auch das Drosselrohr 47 in dem sich verjüngenden Bereich 48 mit Auslassöffnungen 49 ähnlich den Auslassöffnungen 41 des Sprudelungsrohrs 35 versehen, damit die Beruhigung der Wasseroberfläche auch bei dieser Ausführungsform möglich bleibt. Und damit eine Beruhigung der Wasseroberfläche mittels durch die Auslassöffnungen 41 des Sprudelungsrohrs 35 und die Auslassöffnungen 49 des Drosselrohr 47 austretenden Beatmungsgases auch bei axialer Verschiebung des Drosselrohrs 47 möglich bleibt, können diese Auslassöffnungen 49 in nicht dargestellten Weise als in Axialrichtung verlaufende Langlöcher ausgebildet sein, die sich entweder über den gesamten Verschiebungsweg oder nur einen Teil des Verschiebungsweges des Drosselrohrs 47 mit den Auslassöffnungen 41 des Sprudelungsrohrs 35 überlappen. Beispielsweise kann man die Lage und die Länge der Langloch-Auslassöffnungen des Drosselrohrs 47 derart gestalten, dass mit zunehmender Absenkung des Drosselrohrs 47 relativ zum Sprudelungsrohr 35 der Überlappungsbereich mit den Auslassöffnungen 41 des Sprudelungsrohrs 35 abnimmt, somit mit zunehmender Drosselung der Menge des in das Wasser 31 gesprudelten Beatmungsgases die Menge des durch die Auslassöffnungen 41 und 49 auf die Wasseroberfläche geblasenen Beatmungsgases entsprechend reduziert wird, entsprechend dem mit einer solchen zunehmenden Drosselung geringer werdenden Bedarf an Beruhigung der Wasseroberfläche. Eine derartige Koppelung des Maßes der Beruhigung der Wasseroberfläche an das Maß der Drosselung der Besprudelung des Wassers 31 mit Beatmungsgas kann dadurch verfeinert werden, dass man auch die Auslassöffnungen 41 des Sprudelungsrohrs 35 als sich in Axialrichtung erstreckende Langlöcher ausbildet, wodurch die wählbare Überlappung der Auslassöffnungen 49 mit den Auslassöffnungen 41 über einen größeren Bereich möglich ist.

Figur 7 zeigt eine Ausführungsform einer bei der Beatmungsgaskonditionierungseinrichtung 13 gemäß Figur 6 verwendbaren Kombination von Sprudelungsrohr 35 und Drosselrohr 47 in vergrößerter Einzeldarstellung. Wie bei den Ausführungsformen gemäß Figuren 3 und 5 werden auch bei den Ausführungsformen gemäß Figuren 6 und 7 die Diffusionsöffnungen 39 durch Maschen einer Maschenware 51 gebildet, die aus den gleichen Materialien bestehen kann, wie sie schon im Zusammenhang mit Figur 3 erwähnt worden sind. Es besteht nun die in Figur 6 erkennbare Möglichkeit, den Rohrendbereich 38 des Sprudelungsrohrs 35 sich praktisch bis hinab zur Heizplatte 33 erstrecken zu lassen und die Maschenware 51 auf der Innenwand des Rohrendbereichs 38 anzuordnen, wobei dieser Rohrendbereich mit (nicht gezeigten) großflächigen Wanddurchgangsöffnungen versehen ist, um einen weitgehend ungehinderten Zugang des Beatmungsgases zu der Maschenware 51 zu ermöglichen. Eine andere, in den Figuren 3 und 5 bis 7 gezeigte Möglichkeit besteht darin, den eigentlichen Rohrteil des Sprudelungsrohrs 35 am ins Wasser 31 getauchten Ende zu verkürzen und an dieses Ende eine Sprudelungsanordnung anzusetzen, aufweisend einen Trägerteil 53 mit einem Trägerboden 55, einer in den Figuren 6 und 7 nicht sichtbaren Trägerdecke und einem den Trägerboden 55 und die Trägerdecke verbindenden, sich in Axialrichtung erstreckenden Trägersteg 57 und eine vom Trägerteil 53 in Hohlzylinderform gehaltene Maschenware 51.

Insbesondere bei einer Ausführungsform, bei welcher das eigentliche Rohr des Sprudelungsrohrs 35 nicht bis hinab zu Heizplatte 33 reicht sondern an das untere Ende des eigentlichen Rohrs das Trägerteil 53 mit Maschenware 51 angesetzt ist, empfiehlt es sich, für das Trägerteil 53 ein Material guter Wärmeleitfähigkeit zu wählen, beispielsweise Metall, um nun eine Erwärmung des Rohres mit Heizenergie der Heizplatte 33 zu ermöglichen.

Mit einer erfindungsgemäßen Beatmungsgaskonditionierungseinrichtung lassen sich mit relativ geringem technischem Zusatzaufwand gegenüber herkömmlichen Beatmungsgaskonditionierungseinrichtungen, insbesondere der quasi-genormten Ausführungsform als Einwegprodukt, auch bei hohem Beatmungsgasdurchfluss eine optimale Konditionierung und somit eine optimale Aufbereitung des Beatmungsgases auf eine Temperatur von 37°C und eine Luftfeuchtigkeit von etwa 100% (44mg/l Luft) erreichen.

## Patentansprüche

1. Sprudelungsrohr, ausgebildet für das Eintauchen in Flüssigkeit (31), welche in eine Konditionierungskammer (29) einer Beatmungsgaskonditionierungseinrichtung (13) gefüllt ist, wobei das Sprudelungsrohr (35) in einem Eintauchbereich mit mindestens einer Diffusionsöffnung (39) versehen ist, die zur Ausleitung von Beatmungsgas, welches einer Einlassseite des Sprudelungsrohrs (35) zugeführt wird, in die Flüssigkeit (31) ausgebildet ist.

2. Sprudelungsrohr nach Anspruch 1, welches an einem auslassseitigen Rohrende (40) geschlossen ist und eine Mehrzahl von um dessen Rohrumfang verteilte Diffusionsöffnungen (39) aufweist, die in mindestens einer radialen Ebene um den Umfang des Sprudelungsrohrs (35) verteilt ist.

3. Sprudelungsrohr nach Anspruch 2, dessen Diffusionsöffnungen (39) von Rohrwanddurchgangsöffnungen eines Rohrbereichs des Sprudelungsrohrs (35) oder durch Maschenöffnungen eine in Rohrform gehaltenen Maschenware (51) gebildet sind.

4. Sprudelungsrohr nach mindestens einem der Ansprüche 1 bis 3, welches in einem axialen Bereich, der sich bei ordnungsgemäßer Flüssigkeitsbefüllung der Konditionierungskammer (29), für welche das Sprudelungsrohr (35) ausgebildet ist, oberhalb der Flüssigkeit befindet, mindestens eine Auslassöffnung (41) aufweist, die vorzugsweise in einem sich in Richtung des auslassseitigen Rohrendes (40) verjüngenden Abschnitt (45) des Sprudelungsrohrs (35) ausgebildet ist

5. Sprudelungsrohr nach mindestens einem der Ansprüche 1 bis 4, das als gerades Rohrstück oder als abgewinkeltes Rohrstück ausgebildet ist.

6. Sprudelungsrohr nach mindestens einem der Ansprüche 1 bis 5, das mit einer Drosselvorrichtung versehen ist, mittels welcher die Menge des in die Flüssigkeit sprudelnden Beatmungsgases gesteuert werden kann.

7. Sprudelungsrohr nach Anspruch 6, aufweisend eine Drossel in Form eines auf die Außenseite des Sprudelungsrohrs (35) aufgesetzten außenseitigen Aufsatzes (47) und/oder in Form eines auf der Innenseite des Sprudelungsrohrs (35) befindlichen innenseitigen Einsatzes, die relativ zu dem mit der mindestens einen Diffusionsöffnung (39) versehenen Teil des Sprudelungsrohrs (35) derart bewegbar ist, dass **dadurch** ein wählbarer Anteil der mindestens einen Sprudelungsöffnung (39) des Sprudelungsrohrs (35) verschlossen werden kann.

8. Beatmungsgaskonditionierungseinrichtung zur Befeuchtung und Erwärmung ihr von einer Beatmungsgasquelle zugeführten und nach der Konditionierung von ihr zur Weiterleitung an einen zu beatmenden Patienten abgegebenen Beatmungsgases, aufweisend:
eine Konditionierungskammer (29), die zur Befüllung mit einer das Beatmungsgas befeuchtenden und erwärmenden Flüssigkeit ausgebildet ist;
einen Kammereinlass (bei 37), der zur Verbindung mit der Beatmungsgasquelle ausgebildet ist;
einen Kammerauslass (bei 43), der zur Verbindung mit dem Patienten ausgebildet ist;
eine in der Konditionierungskammer (29) angeordnete, zur Erwärmung der Flüssigkeit ausgebildete Flüssigkeitsheizeinrichtung; und
eine Sprudelungseinrichtung, die derart ausgebildet und in der Konditionierungskammer (29) derart angeordnet ist, dass sie mindestens einen Teil des von der Beatmungsgasquelle kommenden Beatmungsgases in mindestens einen Teil des Volumens der in der Konditionierungskammer (29) befindlichen Flüssigkeit zur Erwärmung und Befeuchtung des Beatmungsgases mithilfe dieses Flüssigkeitsvolumens hineinsprudelt.

9. Beatmungsgaskonditionierungseinrichtung nach Anspruch 8, deren Flüssigkeitsheizeinrichtung als eine Heizplatte (33) ausgebildet ist, die bei ordnungsgemäßer Flüssigkeitsbefüllung der Konditionierungskammer (29) in die Flüssigkeit eingetaucht ist und für die Flüssigkeit eine Bodenplatte bildet.

10. Beatmungsgaskonditionierungseinrichtung nach Anspruch 8 oder 9, deren Sprudelungseinrichtung beheizbar ist, insbesondere durch Kontakt mit der Flüssigkeitsheizeinrichtung und/oder mittels einer zur Flüssigkeitsheizeinrichtung zusätzlich vorgesehenen Zusatzheizeinrichtung.

11. Beatmungsgaskonditionierungseinrichtung nach mindestens einem der Ansprüche 8 bis 10, deren Sprudelungseinrichtung als Sprudelungsrohr (35) nach mindestens einem der Ansprüche 1 bis 7 ausgebildet ist.

12. Verbindungsschlauch, ausgebildet für die Verbindung einer Einlassseite einer Konditionierungskammer (29) einer Beatmungsgaskonditionierungseinrichtung (11) mit einer Beatmungsgasquelle, wobei ein mit der Einlassseite der Konditionierungskammer (29) verbindbarer Endbereich des Verbindungsschlauchs (17) mit einem Sprudelungsrohr (35) nach mindestens einem der Ansprüche 1 bis 7 versehen ist.

13. Verfahren zur Temperatur- und Feuchte-Konditionierung von einem Patienten zuzuführendem Beatmungsgas durch Wärme- und Feuchtigkeitsaustausch mit einer in eine Konditionierungskammer (29) gefüllten Flüssigkeit, wobei das Beatmungsgas mittels einer Sprudelungseinrichtung in einen unterhalb der Flüssigkeitsoberfläche liegenden Volumenbereich der Flüssigkeit eingesprudelt wird.

14. Verfahren nach Anspruch 13, bei welchem die Einsprudelung des Beatmungsgases in die Flüssigkeit mit einem Sprudelungsrohr (35) nach mindestens einem der Ansprüche1 bis 7 durchgeführt wird.

15. Verfahren nach Anspruch 13 oder 14, bei welchem die Einsprudelung des Beatmungsgases bei einer Beatmungsgaskonditionierungseinrichtung (13) nach mindestens einem der Ansprüche 8 bis 11 durchgeführt wird.
